# EUROPÄISCHE PATENTANMELDUNG

(11) **EP 2 676 700 A2**
(43) Veröffentlichungstag der Anmeldung: **25.12.2013**
(21) Anmeldenummer: 13003131.3
(22) Anmeldetag: 19.06.2013
(51) Int. Cl.: A61N 2/02

(54) **Verfahren und Vorrichtung für das maschinengestützte Beckenbodentraining**

(30) Priorität: 20.06.2012 DE 102012012149
(71) Anmelder: Quantenmedicine AG, 9495 Triesen (LI)
(72) Erfinder: Fischer, Emil-Gerhard, 88079 Kressbronn (DE); Dobler, Karl, 6845 Hohenems (AT); Fritsch, Erwin, 6845 Hohenems (AT)
(74) Vertreter: Riebling, Peter

(57) **Zusammenfassung**

Verfahren für das maschinengestützte Beckenbodentraining der Beckenboden-Muskulatur mit der transpelvinen Magnetstimulation (TPM), bei dem über mindestens eine sitzflächenseitig angeordnete Magnetspule (5) Einzelpulse bis zu 50 Hz in den menschlichen Körper hinein erzeugt werden, um die Beckenbodenmuskulatur zu einer Kontraktion oder Entspannung anzuregen, wobei in einem Zeitraum vor Beginn der transpelvinen Magnetstimulation und mindestens während der transpelvinen Magnetstimulation dem menschlichen Gewebe noch zusätzlich Sauerstoff und/oder Ozon zugeführt wird. Die Behandlung ist invasiv und erfolgt somit in bekleidetem Zustand.

## Beschreibung

Die vorliegende Erfindung betrifft ein Verfahren und eine Vorrichtung zur maschinengestützten nichtinvasiven nicht entkleidenden und kontaktlosen Behandlung biologischen Gewebes mittels elektromagnetischen Feldern, insbesondere mittels Magnetfeldern nach dem Oberbegriff des Patentanspruches 1.

Sie dient vornehmlich zur nichtinvasiven Stimulation von Körpergewebe, insbesondere Muskelgewebe und/oder Nervenzellen. Die Vorrichtung ist dabei insbesondere aber nicht einschränkend für die Behandlung einer Beckenbodenschwäche ausgelegt. Sie ist darüber hinaus für eine Vielzahl prophylaktischer und therapeutischer Anwendungen im physiotherapeutischen, orthopädischen sowie dermatologischen Bereich ausgelegt.

Das physikalische Prinzip der Magnetstimulation ist die elektromagnetische Induktion. Ein Kondensator entlädt sich über eine Magnetspule und erzeugt ein Magnetfeld. Durch die Magnetfeldänderung wird im Körper eine Spannung induziert, aus welcher ein Stromfluss resultiert. Erreicht dieser Strom eine ausreichende Stärke, so werden im Körper u.a. Muskel- und Nervenzellen gereizt.

In der medizinischen Rehabilitation und Sportmedizin ist die Elektrostimulation ein gängiges Verfahren, welches unter anderem auch in der Inkontinenzbehandlung Anwendung findet. Die Elektrostimulation ist jedoch hinsichtlich ihres Einsatzgebietes weitgehend auf die Peripherie bzw. auf die peripheren Strukturen des zu behandelnden Organismus beschränkt.

Wegen des vergleichsweise hohen elektrischen Widerstands von Gewebsstrukturen, wie etwa Haut und Knochen, kommt es innerhalb des Körpergewebes zu einem erheblichen Spannungsabfall. Zur Erzielung therapeutischer Erfolge müssen bei der Elektrostimulation demzufolge hohe Stromstärken bzw. elektrische Spannungen angewendet werden. Dies kann jedoch zu einer schmerzhaften Reizung sensorischer Nervenenden speziell in der Haut führen, die für den Patienten unbedingt zu vermeiden sind. Von daher sind tief unter der Haut liegende Nerven- und Muskelzellen in der Praxis mittels Elektrostimulation kaum zu stimulieren.

Jene Unzulänglichkeiten der Elektrostimulation können mittels elektromagnetischer Induktion weitgehend umgangen werden. Sich zeitlich verändernde elektrische Ströme erzeugen gemäß der Lenz'schen Regel transiente Magnetfelder, welche innerhalb des zu behandelnden Körpergewebes ein der zeitlichen Änderung des magnetischen Feldes proportionales elektrisches Feld nach sich ziehen. Derartige, mittels elektromagnetischer Induktion in tieferliegenden Gewebezonen erzeugten elektromagnetischen Felder können an Zellmembranen des zu behandelnden Gewebes, typischerweise am menschlichen oder tierischen Organismus, eine Potenzialdifferenz hervorrufen, die etwa zu einer Depolarisierung der Nervenzellen führen kann.

Die dabei entstehenden Ionenströme senken typischerweise das Membranpotenzial der benachbarten Neuronen, sodass ein von außen applizierter Anfangsimpuls kettenreaktionsartig bis zur motorischen Endplatte, bzw. bis zur Synapse und zum entsprechenden Muskel weitergeführt wird. Die Folge hiervon ist eine Muskelkontraktion in Abhängigkeit der zeitlichen Veränderung und Amplitude bzw. Intensität des applizierten elektromagnetischen Feldes. Dieses Nerven-Stimulationsprinzip kommt bereits in der transkraniellen Magnetstimulation erfolgreich zur Anwendung. Davon abgeleitet ist die Behandlung in der Zielregion des Beckens eines Patienten. Dort befinden sich eine Vielzahl von gestaffelt und gitterförmig übereinander gelagerten Muskeln, die als Funktionseinheit des Beckenbodens und Sphinkterverschlüssen eine wichtige Rolle bei der Miktion, Defäkation und erektilen Funktion spielen und sich auch auf die Stabilisierung von Rumpf und Becken und die Sicherung der Beckenorgane beziehen. Urologische Therapiekonzepte betonen eine Trainingsnotwendigkeit des Beckenbodens, die entweder aktiv oder durch Stimulation durchzuführen ist.

Dies führt nachweislich zu einer muskulären Adaptation der Zielmuskulatur und verbesserten muskulären Leistungsfähigkeit einschließlich morphologischer Veränderungen, und ist z.B. per Ultraschall nachweisbar. Damit wird nicht nur die Levatorplatte langfristig angehoben, sondern auch der Muskelfaserquerschnitt vergrößert, was zu einer Zunahme der Festigkeit des aktiven und passiven Halteapparats des kleinen Beckens führt. Aufgrund ihrer identischen Faserzusammensetzung lässt sich die Beckenbodenmuskulatur somit identisch zur Skelettmuskulatur der Extremitäten oder des Rumpfs trainieren.

Hieraus ergeben sich Indikationen zur Behandlung der verschiedenen Inkontinenzarten wie z.B. Belastungs- und Dranginkontinenz und ihrer Mischformen, die Stuhlinkontinenz sowie die Inkontinenz nach Prostatektomie. Auch das Pelvic Pain Syndrom, dessen multifaktorielle Verursachung noch nicht vollständig geklärt ist, zählt über die musculosketalen und myofaszialen Einflüsse zum Einsatzgebiet.

Die Belastungsinkontinenz basiert auf einer Insuffizienz des Beckenbodens, der u.a. ein Dammschnitt, eine Muskelüberdehnung durch Schwangerschaft oder Geburt oder ein hormonelles Defizit zugrunde liegen kann. Eine Tonusverminderung und eine unzureichende Reflexantwort können somit einer Druckerhöhung im Bauchraum nicht widerstehen, so dass schon beim Niesen, Lachen oder einfachen Gehen der natürliche Harnverschluss versagt. Via Nervenstimulation wirkt die Transpelvine Magnetstimulation (TPM) als direktes Kontraktionstraining sämtlicher Anteile der Beckenbodenmuskulatur, wobei auch Muskelbereiche angesprochen werden, die mit körperlichen Trainingsübungen, wie aktivem Beckenbodentraining, nicht zu erreichen sind. So kann die Beckenbodenmuskulatur mit einer aktiven isolierten Anspannung nur einen geringen Teil ihrer möglichen Kraft produzieren. Auch ist bei einer TPM - entsprechend den Erfahrungen der Elektrotherapie - die Rekrutierungsreihenfolge der Muskelfasern nicht eindeutig festgelegt und erfolgt damit global.

Wird also mittels TPM ein einzelner Beckenbodenmuskel elektrisch stimuliert, besteht die Tendenz, dass sich auch sämtliche anderen Muskeln der Verbundeinheit kontrahieren.

Aufgrund ihrer engen räumlichen Nähe zum Blasenausgang ist der Verschlussmechanismus des Harntrakts im Falle einer operativen Entfernung der Prostata stark gefährdet Eine radikale Prostatektomie verkürzt nicht nur die Harnröhre, sondern kann auch den Blasenverschluss des Rhabdosphinkters verletzen und gilt auch für das Verfahren der Transurethralen Prostatektomie (TUR). Männer werden damit auf den untrainierten Kontinenzverschluss des Beckenbodens zurückgeworfen. Die Symptomatik ist identisch zu der einer Belastungsinkontinenz, d.h. die Nervenstimulation mittels TPM dient der Kräftigung des muskulären Grundtonus des Beckenbodens und des Trainings der reflektorischen Kontraktionskraft.

Eine Stuhl-Inkontinenz ist eine Störung im komplexen Zusammenspiel zwischen Motorik, Sensorik und anatomischen Gegebenheiten. Werden z.B. die Hauptmuskelzüge des Beckenbodens wie M. sphinkter ani internus und externus, M. puborectalis und levator unzureichend nerval angesprochen, dann äußert sich dies auch in einer verminderten Perzeption der Rektum- und Analsensorik. Eine Muskelstimulation mittels TPM kräftigt damit nicht nur die für eine Kontinenz notwendigen Beckenbodenstrukturen, sondern verbessert auch die für eine Reflexkontraktion notwendige Wahrnehmung einer notwendigen Schließmuskelaktivität. Ferner ist aus der DE 298 08 990 U1 bereits ein Sitz- und Liegemöbel für die Magnetfeldtherapie bekannt, welches eine oder mehrere Applikatorvorrichtungen zur Erzeugung von Magnetfeldern aufweist. Die Applikatorvorrichtung kann dabei in die Polsterung des jeweiligen Sitz- oder Liegemöbels eingebettet sein. Ferner kann die Applikatorvorrichtung zur Erzeugung von Magnetfeldern mit Spulen ausgestattet sein, die eine Ganzkörper- oder eine Teilkörpertherapie mit in Reihen oder kreisförmig angeordneten Vollkern-Metallspulen ermöglicht.

Die therapeutischen und prophylaktischen Auswirkungen elektromagnetischer Felder auf zu behandelndes Körpergewebe können von einer Vielzahl unterschiedlicher Faktoren, insbesondere der konkreten Zusammensetzung des Körpergewebes, etwa seines Wasser- und Fettgehaltes, aber auch von der geometrischen Form, Intensität sowie vom zeitlichen Verlauf der applizierten elektromagnetischen Felder abhängen. Moderne Behandlungsmethoden sehen zudem eine individuell auf den jeweiligen Patienten abgestimmte Anwendung elektromagnetischer Felder vor.

Da zudem die genauen Wirkzusammenhänge sowie die Interaktion von Magnetfeldern mit Körpergewebe, insbesondere mit Nerven- und Muskelzellen noch nicht gänzlich im Detail erforscht sind und somit die Anwendungsparameter für die Magnetfeldtherapie oft nur empirisch ermittelbar sind, ist es besonders wünschenswert, die geometrische Ausdehnung, die räumliche Verteilung sowie die Intensität der elektromagnetischen Felder möglichst gezielt steuern und beeinflussen zu können.

Es ist daher Aufgabe der vorliegenden Erfindung, eine Vorrichtung zur Behandlung biologischen Gewebes mittels Magnetfeldern zur Verfügung zu stellen, die eine möglichst universelle Adaptierbarkeit an vorgegebene Behandlungsschemata, insbesondere eine individuelle Anpassung und Veränderung der räumlichen Ausdehnung, Verteilung, Position sowie der Intensität und des zeitlichen Verlaufs von zu erzeugenden elektromagnetischen Feldern ermöglicht. Daneben soll sich die Vorrichtung durch einen möglichst einfachen, robusten, wenig störanfälligen sowie kostengünstig herstellbaren und für die Erzeugung von ausreichend dimensionierten Magnetfeldern geeigneten Aufbau auszeichnen. Diese Aufgabe wird erfindungsgemäß dadurch gelöst, dass die Vorrichtung zur Behandlung biologischen Gewebes zumindest eine Einrichtung zur Erzeugung wenigstens eines Magnetfeldes aufweist. Die MagnetfeldErzeugungseinrichtung weist dabei zumindest eine mit einem elektrischen Strom beaufschlagbare Spule und zumindest einen mit der Spule induktiv zusammenwirkenden Kern aus einem magnetisierbaren Material auf. Der Kern besteht aus einem magnetisierbaren Material und bewirkt eine Verstärkung und/oder eine geometrische Beeinflussung des von der Spule erzeugbaren Magnetfeldes.

Über Afferenzen des N. pudendus erfolgt gleichzeitig ein mono- und polysynaptischer Reflex mit konsekutiver Anspannung der Beckenbodenmuskulatur. Der Reflexbogen entsteht dabei nach dem Prinzip der Weiterleitung sensorischer Reize zum Rückenmark, wobei Interneurone erregt und deren Schutzreaktion mit einem efferenten Nervenimpuls als Kontraktionssignal an die Beckenbodenmuskulatur weitergeben werden können.

Bei der Dranginkontinenz besteht eine Übersensibilisierung von Rezeptoren, die in der Schleimhaut der Harnblase den Füllungszustand kontrollieren und bereits bei kleinsten Harnblasenvolumina einen starken Harndrang suggerieren.

Das Gehirn reagiert daraufhin mit einer nicht unterdrückbaren Kontraktion der Harnblasenmuskulatur (detrusor vesicae), die in einer unwillkürlichen Öffnung des Blasensphinkters (Rhabdosphinkter) resultiert. Die hemmende Wirkung der TPM erfolgt nach Erkenntnissen der Elektrostimulation entsprechend tierexperimenteller und klinischer Studien über eine Aktivierung der Afferenzen des N. pudendus. Diese erfolgt bei geringer Blasenfüllung durch eine Erregung des N. hypogastricus, ansonsten durch eine direkte Hemmung der Kerne des N. pelvicus im Sakralmark und auch über eine supraspinale Hemmung des Detrusorreflexes. Die entsprechende Reizfrequenz liegt zwischen 5 - 10 Hz. Auch bei der Dranginkontinenz ist ein intakter Beckenboden von entscheidender Bedeutung. Kommt es zur unwillkürlichen Öffnung des Rhabdosphinkters, wird die Harnentleerung über eine Reflexkontraktion der schnellen Muskelfaseranteile des Beckenbodens gehemmt.

Zur Magnetstimulation der verschiedenen Arten der Harninkontinenz existieren über ein Vorläufersystem über 70 Studien. Nach einer Behandlungsfrequenz von 16-20 Anwendungen zwischen 6-8 Wochen ist in 41 - 78 % der Behandlungsfälle eine Remission, Symptombefreiung oder deutliche Verbesserung zu erwarten. Die Erhaltungsdauer liegt bei durchschnittlich 6-12 Monaten, wobei im Einzelfall auch 35 Monate erreicht wurden. Das Ergebnis einer Evidenzanalyse bezieht sich auf eine Gesamtstichprobe von 3028 Patienten. Bei 50 Studien Urinary incontinence, 1 Fecal incontinence, 1 intersititiel cystitis, 1 Pelvic-Pain-Syndrom, 3 gesunde Probanden, 1 Tierexperiment, 10 Overactive-Bladder-Syndrom liegen die EbM-0-Level (Einstufung nach Cochran Collaboration) bei 1 Studie, (EbMO = Experimentelle Arbeit), EbM1a = 1 Studie, EbM1b = 11 Studien, EbM2a = 5 Studien, EbM2b = 9, EbM2c = 12, EbM3b = 12, EbM3a = 0, EbM4 = 23, EbM5 = 1 Studie.

Zwar sind viele Studien ohne Vergleichsgruppen und es ist auch bei nur etwas 12 % der Studien ein 1b-Qualifizierung (RCT = Randomized Controlled Trial) gerechtfertigt, aber allein aufgrund der hohen Zahl unabhängiger Forscher aus vielen Regionen der Welt ist selbst den Studien mit der Klassifikation 4 ein wichtiger Stellenwert einzuräumen. Hinsichtlich der Therapiequalität wurde 11 x die Note 1 vergeben, einmal die Note 1-2, 20 x die Note 2, 21 x die Note 2-3 bzw. 3, 7 x die Note 3-4 bzw. 4, und 2 x die Note 5. D.h. dass gut 50 % der Therapieergebnisse die Bewertung gut / sehr gut, knapp 5 % die Bewertung mangelhaft und knapp 45 % die Bewertung befriedigend / ausreichend erhalten haben. Im Mittel ist die Therapiequalität damit mit 2,4 bewertet.

Die Ätiologie des Pelvic Pain Syndroms ist noch nicht vollständig geklärt. Für die Unterbauchschmerzen dürften sowohl viszeral-neuronale als auch myofaszial-muskulo-skeletale Strukturen verantwortlich sein. Gerade bei der myofaszialen, also der die Muskelansatzpunkte und das Bindegewebe des Muskels betreffenden Schmerzentstehung, scheinen lokale Verhärtungen in den Beckenbodenmuskeln eine entscheidende Rolle zu spielen. Ähnlich wie bei den Schmerzsyndromen des Bewegungsapparats kommt es dabei zu Nervenquetschungen und entsprechenden Schmerzsensationen. Ein TPM Einsatz führt zu einer Steigerung der Mikrozirkulation im Beckenraum, die über Muskelkontraktionen des Beckenbodens zustande kommt. Da Muskelverhärtungen immer auch auf einer Einschränkung der Durchblutung basieren, lösen sich diese bei beginnender Blutrefundierung wieder auf.

Die Beckenbodenmuskulatur spielt bei der Entstehung und dem Aufrechterhalten einer Erektion und auch bei der Orgasmusqualität eine wichtige Rolle. Der M. ischiocavernosus übt z.B. auf die Arteria profunda penis eine pumpende Funktion aus und verhindert über eine Kompression des Corpus cavernosum penis den venösen Blutabfluss (V. profunda penis). Der M. bulbospongiosis wiederum umgibt den Harnröhrenschwellkörper und gerät beim Orgasmus in eine rhythmische Kontraktion. Außerdem löst er eine pulsierende Druckwelle auf den Harnröhrenschwellkörper aus und unterstützt damit die Ejakulation. Der dem Bulbospongiosus vergleichbare M. constrictor vulvae bei der Frau ist der Schließmuskel der Vulva und wird bei der Geburt meistens im Zuge eines Dammschnitts verletzt bzw. durchtrennt. Die Folge ist, dass die für einen Orgasmus notwendigen rhythmischen Konvulsionen nicht mehr ausreichend möglich sind.

Ein Beckenbodentraining bei Patienten mit Erektiler Dysfunktion (ED) bei nachgewiesenem venösen Leck ermöglichte 42 - 47 % der Männer wieder eine zufriedenstellende Erektion bzw. verbesserte bei weiteren 24 % die Impotenzsymptomatik. In einer weiteren Studie zur Wirkung einer speziellen Beckenbodengymnastik bei ED mit leichtem oder mittelschwerem Leck wurde die Erektionsfähigkeit bei 80 % verbessert gegenüber der Sildenafil-Gruppe ("Viagra") mit 74 % und Placebo mit 18 %. Da die Effektivität eines aktiven Beckenbodentrainings grundsätzlich weit über der eines passiven Beckenbodentrainings liegt (18 Therapiesitzungen mit TPM entsprechen etwa 4 Monaten bzw. 4 x 30 Tage x 10 Übungen pro Tag = 1 200 aktiven Übungen), ist eine Übertragung der Ergebnisse uneingeschränkt möglich. Die TPM vermittelte Stimulation der Beckenbodenmuskulatur basiert auf Grundsätzen des allgemeinen Kraft- und Ausdauertrainings sowie Koordination und Sensorik. Dysfunktionen des Diaphragma pelvis (Beckenboden) basieren grundsätzlich auf einer Reduktion von Kraft und Koordination. Ein übungsbedingter Kraftzuwachs resultiert in der Regel aus einer verbesserten Koordination, d.h. der Erhöhung der Anzahl der angesprochenen Muskeifasern. Sie kann aber auch die Koordination größerer Struktureinheiten im Sinne eines synergistischen Zusammenspiels intrinsischer und extrinsischer Muskeln bestehen. Es wird allgemein angenommen, dass synergistische Co-Kontraktionen ein natürliches Reaktionsmuster darstellen, damit plötzlichen abdominellen Druckanstiegen wirksam begegnet werden kann.

Ein Koordinationsvorteil entsteht über die Wiederholung motorischer Impulse, die hier mittels Magnetstimulation die gesamte Beckenbodenmuskulatur umfassen. Dabei macht es keinen Unterschied, ob sich das Training auf eine konzentrische oder isometrische Kontraktion bezieht. Aktive Kontraktionen führen nicht per se zu einer synergistischen Kontraktion. Während also eine willkürliche Innervation einzelne Muskelanteile unabhängig voneinander zusammenziehen lässt, führt die elektrische Reizung eines einzelnen Beckenbodenmuskels zur Massenkontraktion.

Entsprechend der allgemeinen Qualitätsverteilung der Skelettmuskulatur bildet sich die paraurethrale Beckenbodenmuskulatur zu 70 % aus Slow-Twitch-Fibers vom Typ I, also in sog. rote Muskeln, die reich an Myoglobin und gut durchblutet sind und wegen einer bevorzugt aeroben Energieherstellung nur langsam ermüden und zu 30 % in Fast-Twitch-Fibers Typ II sog. Weiße Muskeln, die reich an Glykogen und einer schlechten Durchblutung sehr schnell reagieren. Diese Prädominanz von Typ I beweist ihre vorrangige Stützfunktionsrolle für Organe und neurovaskuläre Strukturen sowie des Ruhedrucks der Urethra. Mit einer Kontraktionsfrequenz von etwa 10 Hz können diese Muskeln den Ruhetonus über einen längeren Zeitraum aufrechterhalten. Stattdessen entwickeln die Fast-Twich-Fibers mit einer natürlichen Kontraktionsfrequenz von bis zu 50 Hz ein eine deutliche höhere Kraft, ermüden jedoch sehr schnell. Sie tragen die Verantwortung, dass die Kontinenz während akuter Belastungssituationen aufrecht erhalten werden kann.

Die Reizkonfiguration des TPM-Systems richtet sich hinsichtlich der Behandlung einer Belastungsinkontinenz bevorzugt nach den schnellen Muskelanteilen und erfolgt gezielt über die Frequenzsteuerung in einem Bereich oberhalb von 30 - 50 Hz. 30 Hz entsprechen auch dem physiologischen Tremor der Muskulatur. Dies erfolgt nach dem sog. Funktionsnachahmungsprinzip, d.h. die Anzahl der Impulse richtet sich nach der Anzahl der natürlichen Aktionspotentiale.

Die eigentlichen stimulationsbedingten Wachstumsreize entstehen durch übungsbedingte Mikrotraumata, also winzigen Muskelrissen. Diese aktivieren das Peptid IGF-1, welches als Effektorhormon in der Wirkkaskade des Human Growth Hormone HGH fungiert und starke anabole Eigenschaften besitzt.

Durch die notwendige Steigerung der Proteinsynthese werden Satellitenzellen stimuliert, die als muskuläre Stammzellen in Lage sind, mit der Muskelzelle zu fusionieren. Um das gleichzeitige Auftreten ubiquitärer Stützproteine, die die Muskelzelle vor Überlastung schützen sollen, um somit eine Verlangsamung der Muskelhypertrophie zu umgehen, sehen die Anwendungsempfehlungen ein leicht progressives Training mittels moderater Erhöhung der Flussdichte vor.

Untersuchungen am Karolinska-Institut in Stockholm beweisen ferner, dass sich langsame Typ I - Fasern trainings- und reizkonfigurationsbedingt in mittelschnelle Fasern von Typ II a umwandeln lassen und umgekehrt. Diese Umwandlung lässt sich damit folgerichtig mit einer vergleichbaren Elektrostimulation des Beckenbodens histologisch nachweisen.

Eine TPM führt nicht nur zu einer Verbesserung der Muskelfaserkoordination, Reflexantwort und Muskelhypertrophie, sondern auch zu einer Durchblutungssteigerung um das Mehrfache des Ausgangswerts. Auch ist eine Neubildung von Blutgefäßen dokumentiert.

Im Hinblick auf eine möglichst maximale Verbesserung von Muskeltonus- und Reflexen innerhalb einer therapeutisch vertretbaren Zeit ist ein alleiniges Koordinations- und Kontraktionstraining der Beckenbodenmuskulatur nur bedingt geeignet. Neuere Erkenntnisse aus der adjuvanten Sportphysiologie legen nahe, noch weitere, die allgemeinen Versorgungs- und Leistungsparameter eines Probanden betreffende Stimulationsverfahren einzusetzen, die via Sauerstoff, Licht und Second- Messenger- Aktivierung die Mikrozirkulation und den Kraft- und Energiezuwachs nachhaltig unterstützen.

Ein wichtiger Begleiteffekt der TPM ist die Aktivierung des Fettstoffwechsels mit einem Abbau von Depotfetten. Dies wirkt sich besonders auf eine Cellulite aus. Obwohl diese hauptsächlich über vertikal verlaufende Bindegewebszüge zustande kommt, besteht die einzige wirksame Therapie in einer Durchblutungssteigerung bei gleichzeitigem Muskeltraining. Damit wird eine Cellulite nicht grundsätzlich korrigiert. Allerdings trägt die Reduzierung von Fettdepots des Unterhautfettgewebes aber regelmäßig zu einer optisch verbesserten Silhouette der Hüft-, Schenkel- und Gesäßkonturen bei.

Eine verbesserte Mikrozirkulation im Beckenraum dient auch indirekt der Prophylaxe von Hüftkopfnekrosen und Hüftgelenksarthrosen, da die Tiefenwirkung einer TPM nicht nur den Beckenboden umfasst, sondern die Gelenksstrukturen in diesem Bereich. Nach neuen Erkenntnissen sind nicht traumatisch bedingte Arthrosen nicht über eine vermehrte Beanspruchung, sondern über eine Minderdurchblutung der Gelenkkapseln bedingt. Dies führt regelmäßig zu einer Abnahme der Knorpelernährung und einer Störung zwischen den abbauenden und aufbauenden Kräften des Knorpels. Eine TPM verbessert nicht nur die Durchblutung und Knorpelernährungssitutation, sondern führt über eine Muskelkontraktion im Hüftbereich zu einem Stimulationsdruck auf das Knorpelgewebe.

Mit der transpelvinen Magnetstimulation TPM wurde eine wirksame Therapieoption in der Behandlung von Beckenbodenmuskel relevanten Dysfunktionsbilder wie der Harninkontinenz, Stuhlinkontinenz, erektile Dysfunktion, Menstruationsbeschwerden, Libido Dysfunktion, Knochen oder Knorpeldysfunktion in Gelenken bis hin zur reproduktiven und präventiven Beckenbodengesundheit entwickelt.

Kernpunkt der Magnetfeldstimulation TPM ist, dass bei dem zu Behandelnden Körperbereich mindestens eine hochwirksame Magnetspule angeordnet wird welche in der Lage ist, ein wirksames Magnetfeld mit Pulsen bis zu 50Hz auf den entsprechenden Körperteil des zu Behandelnden zu übertragen.

Bei der Magnetfeldstimulation TPM werden über mindestens eine Magnetspule Einzelpulse bis zu 50 Hz in den menschlichen Körper erzeugt, um die Beckenbodenmuskulatur zu einer wirksamen Kontraktion oder Entspannung zu bringen. Dadurch wird die Beckenbodenmuskulatur trainiert.

Die hochwirksamen Magnetfeldlinien wirken in einer magnetischen Flussdichte von bis zu einem Tesla. Die Feldstärke, Puls- und Pausezeiten, Frequenzen und Pulsformen sind in Abhängigkeit von bestimmten physiologischen Parametern einstellbar ausgebildet.

Die angewendeten Magnetfelder bringen die Muskulatur des Beckenbodens zu einer starken Kontraktion (M. levator ani), die weit über den Möglichkeiten eines aktiven willkürlichen Beckenbodentrainings liegt. Die Folge sind gute Heilungs- und Besserungsraten, die schon nach einigen Behandlungen zu erreichen sind.

Für ein optimales Training und Aufbau der Muskulatur benötigt diese Sauerstoff. Eine Unterversorgung mit Sauerstoff während eines intensiven Muskeltrainings hinterlässt negative Effekte. Die Zuführung von Sauerstoff während des Muskeltrainings erhöht den Trainings- und Therapieeffekt massiv.

Der Erfindung liegt deshalb die Aufgabe zugrunde, ein Verfahren und eine Vorrichtung für das maschinengestützte Beckenbodentraining mit der transpelvinen Magnetstimulation TPM so auszubilden, daß während der Durchführung der transpelvinen Magnetstimulation eine ausreichende Sauerstoffversorgung zur Verfügung steht. Neben der Zufuhr von Sauerstoff in den Kreislauf des Patienten kann auch Ozon verwendet werden

Zur Lösung der gestellten Aufgabe ist die Erfindung durch die technische Lehre des Anspruches 1 gekennzeichnet.

In einer bevorzugten Ausgestaltung der Erfindung erfolgt die Sauerstoffzugabe über eine Atem- oder Nasenmaske, die dem Patienten einen erhöhten Sauerstoffanteil in der Atemluft zuführt. Durch diese Sauerstoffanreicherung im Blut werden die Muskeln bei deren Trainingsarbeit gut mit Sauerstoff versorgt. Hierbei ist wichtig, dass die Sauerstoffzufuhr so rechtzeitig erfolgt, dass das mit Sauerstoff gesättigte Blut rechtzeitig vor Trainingsbeginn beim Muskel ankommt. Wesentliches Merkmal der Erfindung ist, dass in einem Zeitraum vor Beginn-, während- und auch nach der transpelvinen Magnetstimulation dem menschlichen Gewebe zusätzlicher Sauerstoff zugeführt wird.

Insbesondere wird es bevorzugt, wenn die Zufuhr der Sauerstoffmenge und somit der Sauerstoffanteil in der Atemluft geregelt wird. Die Regelung des Sauerstoffanteils in der zugeführten Atemluft kann auf Messung der Sauerstoffsättigung im Blut basieren. Die Sauerstoffsättigung kann z.B. mit Hilfe eines nicht invasiven Pulsoximeters z.B. in Form einer Klammer an der Fingerspitze oder einer anderen Extremität angebracht werden. Die Regelung des Sauerstoffanteils in der zugeführten Atemluft kann aber auch am CO² Anteil in der ausgeatmeten Luft erfolgen.

Nach einer weiteren Maßnahme der Erfindung ist es vorgesehen, dass zur weiteren Steigerung der beckenbodenseitigen Durchblutung dem Beckenbodenbereich zusätzlich Wärmeenergie zugeführt wird, sodass kein kalter Muskel trainiert wird. Eine solche extern zugeführte Erwärmung steigert die Muskeldurchblutung und kann z.B. durch eine Sitzheizung im Behandlungsstuhl erfolgen. Eine solche Sitzheizung ist bevorzugt als elektrisches Heizkissen ausgebildet. In einer anderen Ausgestaltung ist es vorgesehen, dass ein Wärmeeintrag in die tieferen Beckenbodenbereiche mit einer IR-Strahlung, bevorzugt über eine IR-Strahlungslampe erfolgt.

Es hat sich herausgestellt, dass eine Erwärmung des Muskels (M. levator ani) und eine entsprechende Stimulation des Nervengewebes des Muskels dann besonders wirksam erfolgt, wenn noch ein weiteres Magnetfeld auf die Beckenbodenmuskulatur wirkt, welches unabhängig von dem erstgenannten, im Sitzbereich angeordneten Magnetfeld wirkt.

Nach der Erfindung handelt es sich demnach um zwei einander sich überlagernde Magnetfelder, wobei bevorzugt ist, wenn die Magnetspulen-Anordnung zur Erzeugung des zweiten Magnetfelds (später QRS genannt) im Rückenlehnenbereich des Behandlungsstuhls angebracht ist. Hierauf ist die Erfindung jedoch nicht beschränkt. Es kann auch vorgesehen sein, dass die Magnetspulen-Anordnung für das zweite Magnetfeld teilweise im Rückenlehnenbereich und teilweise im Sitzbereich oder nur im Sitzbereich angeordnet ist.

Ebenso ist die Erfindung nicht auf die Anordnung einer einzigen Magnetspule sowohl für das erstgenannte, im Sitzbereich erzeugte Magnetfeld (Pelvi) beschränkt und ebenso wenig auch auf eine einzige Magnetspule für das zweite Magnetfeld (QRS).

Solche sich überlagernden Magnetfelder können auch durch eine Anzahl von nebeneinander oder übereinander oder koaxial zueinander angeordneten Magnetfeldspulen erreicht werden. Lediglich der einfacheren Beschreibung wegen wird in der folgenden Abhandlung von einer einzigen Magnetspule für das QRS-Magnetfeld und von einer einzigen Magnetspule für das Pelvi-Magnetfeld ausgegangen, obwohl die Erfindung hierauf nicht beschränkt ist.

Bei der Einwirkung des zweiten (z.B. rückenlehnenseitigen) Magnetfeldes in Überlagerung zum ersten (z.B. sitzflächenseitigen) Magnetfeld hat es sich herausgestellt, dass hierbei eine zusätzliche Reizung der Nervenbahnen im Bereich der Beckenbodenmuskulatur entsteht, insbesondere dann, wenn dieses zweite Magnetfeld mit einer Frequenz von etwa 0,5 bis 1.000 Hz wirkt und in seiner Stärke etwa nur im Bereich zwischen 1/100 bis 1/1000stel der Stärke des im Sitzbereich angeordneten sitzflächenseitigen Pelvi-Magnetfeldes liegt. Eine solche geringe Magnetfeldstärke reicht aus, obwohl die Erfindung selbstverständlich auch höhere Magnetfeldstärken vorsehen kann.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.

Alle in den Unterlagen, einschließlich der Zusammenfassung offenbarten Angaben und Merkmale, insbesondere die in den Zeichnungen dargestellte räumliche Ausbildung, werden als erfindungswesentlich beansprucht, soweit sie einzeln oder in Kombination gegenüber dem Stand der Technik neu sind.

Im Folgenden wird die Erfindung anhand von lediglich einen Ausführungsweg darstellenden Zeichnungen näher erläutert. Hierbei gehen aus den Zeichnungen und ihrer Beschreibung weitere erfindungswesentliche Merkmale und Vorteile der Erfindung hervor.

Es zeigen:
- Figur 1:: schematisiert die Darstellung eines Behandlungsstuhls nach der Erfindung
- Figur 2:: ein Diagramm über die Sauerstoffaufnahme im Gewebe in Abhängigkeit von der Einwirkung eines Magnetfeldes über die Zeit
- Figur 3:: die Zufuhr von Sauerstoff in das Gewebe in Abhängigkeit von der Magnetfeldbehandlung
- Figur 4:: schematisiert die Überlagerung von zwei Magnetfeldern im Bereich der Beckenbodenmuskulatur
- Figur 5:: die Vorderansicht der Magnetfeldstruktur nach Figur 4
- Figur 6:: schematisiert die Darstellung der Stimulation eines Muskelstrangs über die transpelvine Magnetstimulation unter Einwirkung eines zweiten im Winkel hierzu gerichteten Magnetfeldes

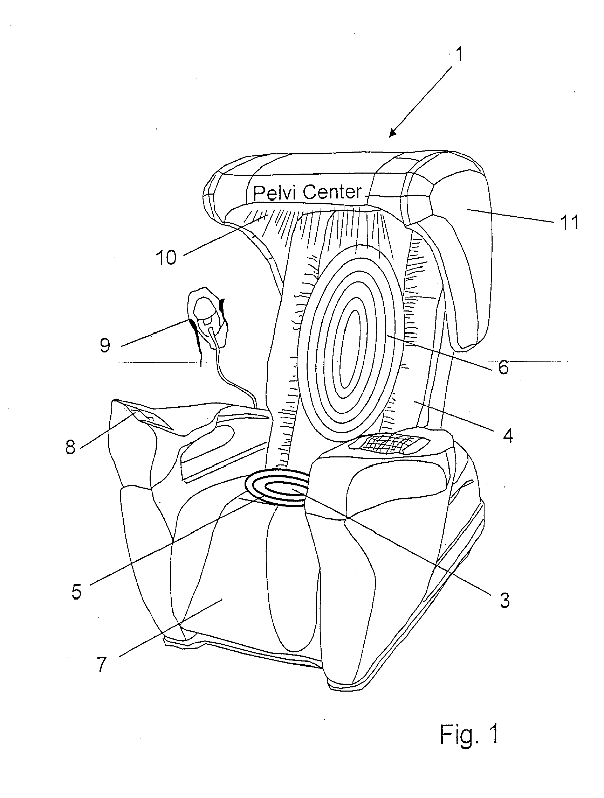

In Figur 1 ist ein Behandlungsstuhl 1 dargestellt, der aus einem kippbaren Kopfteil 11 besteht, in dessen oberen Kopfbereich ein oder mehrere Leuchteneinheiten 10 für die Durchführung einer optisch wahrnehmbaren Lichttherapie angeordnet sind.

In der kippbaren Rückenlehne 4 ist die zweite Magnetspule 6 (QRS) angeordnet, die sich im gezeigten Ausführungsbeispiel über den unteren Rückenbereich der Rückenlehne 4 erstreckt.

Es ist nicht zeichnerisch dargestellt, dass sich diese Magnetspule 6 auch teilweise in den Sitzbereich des Sitzteils 3 hinein erstrecken kann.

Das Sitzteil 3 ist in horizontaler Ebene verschiebbar ausgebildet, d. h. es kann nach vorne und hinten verschoben werden, und zusätzlich ist am vorderen Ende des Sitzteils 3 ein kippbares Fußteil 7 angeordnet.

Auf den Seitenteilen sind Armlehnen angebracht, auf denen jeweils Bedieneinheiten 8 angeordnet sind.

Wichtig ist, dass im Sitzbereich (Sitzteil 3) eine für die transpelvine Magnetstimulation geeignete Magnetspule 5 (Pelvi) angeordnet ist, die das geforderte Beckenbodentraining durchführt.

Die durch die Einwirkung der starken Magnetfeldänderungen bei der TPM ausgelöste elektrische Potentialänderung in der Beckenboden-Muskulatur bewirkt eine Depolarisation von Neuronen mit Auslösung von Aktionspotentialen in der Muskulatur. Die Stärke dieses elektrischen Feldes fällt mit der Entfernung von der Spule 5 in erster Näherung exponentiell ab und hängt von den Eigenschaften des Kondensatorstromes und der Spule 5 ab. Die Stromstärke in der Spule kann mehrere 100 Ampere erreichen. Verwendet werden sogenannte Ein- oder Mehrfachspulen unterschiedlicher Form und Anordnung, wobei vereinfacht von einer Rundspule gesprochen wird. Die verschiedenen Spulen können sich jeweils ganz oder teilweise überlagern, wodurch sich auch das Magnetfeld überlagert.

Wichtig ist ferner, dass eine Sauerstoffmaske 9 vorhanden ist, die über einen Schlauch mit einem Sauerstoffgenerator verbunden ist, so dass beim Anlegen der Sauerstoffmaske 9 eine wirksame Sauerstoffzufuhr über die Atemluft in das Gewebe erfolgt.

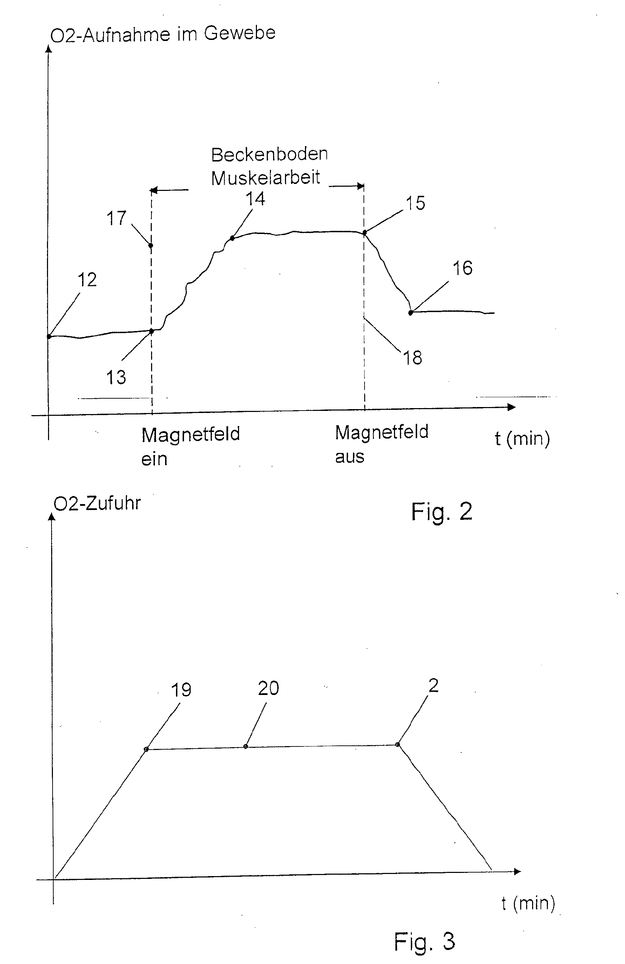

In Figur 2 ist ein solcher Behandlungsverlauf schematisiert dargestellt. Es ist erkennbar, dass die TPM-Behandlung bei Position 12 beginnt und bei Position 13 das Pelvi-Magnetfeld der Magnetspule 5 eingeschaltet wird. Dadurch wird der Sauerstoffbedarf im Gewebe stark erhöht. Bei Position 13 wird das Magnetfeld eingeschaltet und bei Position 18 ausgeschaltet. In diesem Bereich wird eine starke Muskelarbeit der Beckenbodenmuskulatur mit einem entsprechend erhöhten Sauerstoffbedarf induziert, der durch die erfindungsgemäße Zufuhr von separatem Sauerstoff über die Atemluft kompensiert werden muss.

Der Sauerstoffbedarf ist bei Position 14 auf seinem Maximum und hält bis zur Position 15 an. Bei Position 18 wird das Magnetfeld abgeschaltet. Der Sauerstoffbedarf verringert sich dann von Position 15 bis zur Position 16.

Um im Bereich zwischen den Positionen 13 und 18 dem erhöhten Sauerstoffbedarf der Muskulatur im Beckenboden zu entsprechen, zeigt die Figur 3, dass eine gewisse Zeit vor Einschaltung des Magnetfeldes (zum Beispiel bereits bei Position 12) der Sauerstoffgehalt im Gewebe bei Position 19 einen maximalen Bereich erreicht hat und dann während der gesamten Behandlungsdauer 20 maximal Sauerstoff dem Gewebe zugeführt wird, um bei der Position 2 wieder abgeschaltet zu werden.

Wichtig ist, dass im Bereich zwischen den Positionen 13 und 18 während der TPM-Muskelstimulation die atemgestützte Sauerstoffzufuhr maximal ist, um die Beckenbodenmuskulatur mit ausreichend Sauerstoff als Schutz gegen Beschädigung zu schützen. Hierbei kann die Sauerstoffzufuhr über die Atemwege bereits schon einige Minuten vor Einleiten der TPM-Stimulation bei Position 13 beginnen.

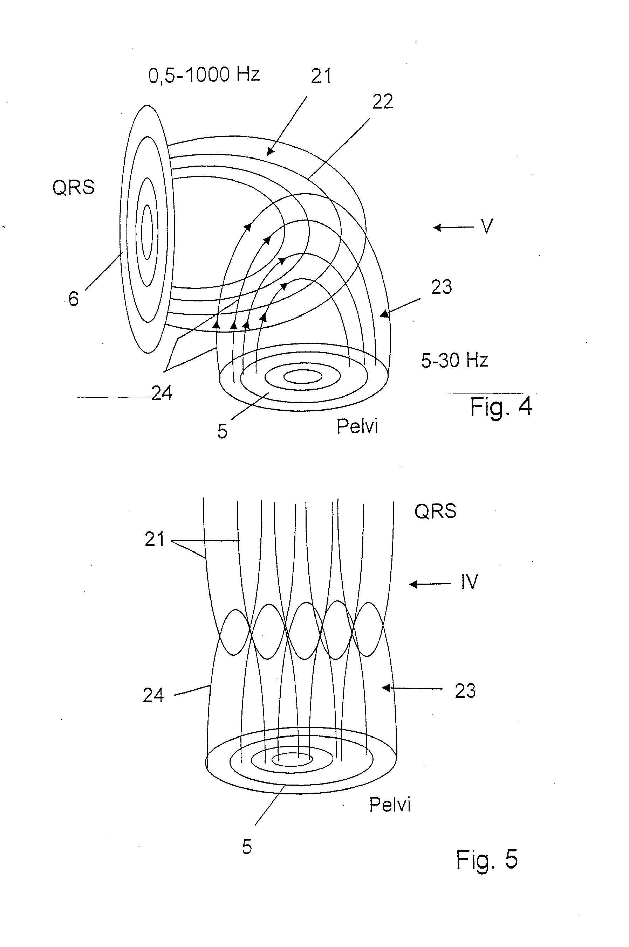

Die Figur 4 zeigt, dass die Zufuhr von Sauerstoff in die Beckenbodenmuskulatur noch mit einer zusätzlichen Stimulation des Nervensystems im Bereich der Beckenbodenmuskulatur durch das zweite rückenlehnenseitige Magnetfeld QRS mit der zusätzlichen Magnetspule 6 kombiniert wird.

Die zusätzliche rückenlehnenseitige Magnetspule 6 erzeugt im Winkel zur sitzflächenseitigen Magnetspule 5 ein zusätzliches Magnetfeld 21 mit höherer Frequenz aber geringerer Intensität, so dass sich die Feldlinien 22 des Magnetfeldes 21 der Magnetspule 6 dem Magnetfeld 23 der Magnetspule 5 und dessen Feldlinien 24 überlagern, sich gegenseitig schneiden und verstärken.

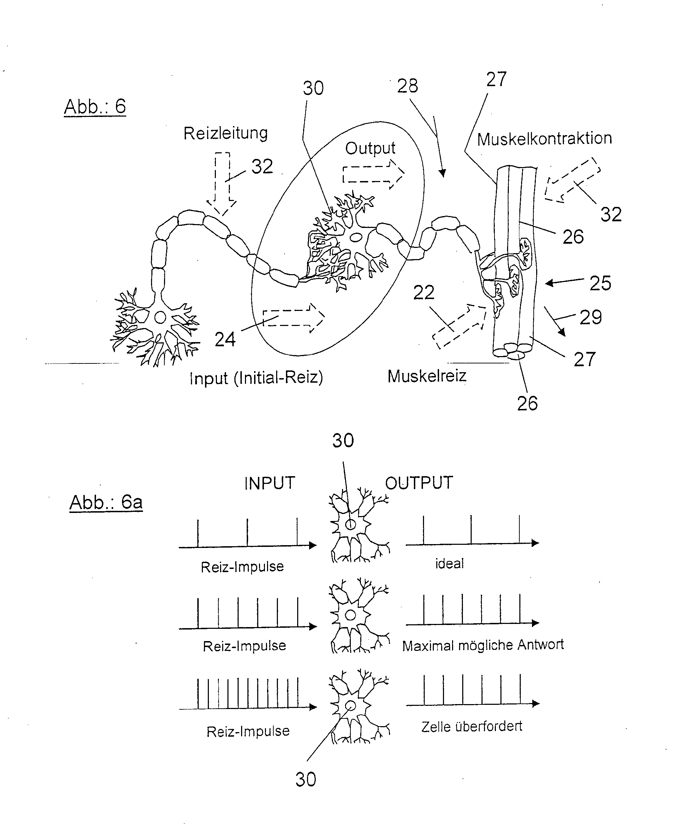

Dies ist in Figur 5 in Frontalansicht zu Figur 4 dargestellt, wo gezeigt ist, dass sich die schwächeren Feldlinien 22 des Magnetfeldes 21 mit den stärkeren Feldlinien 24 des Magnetfeldes 23 kreuzen, überschneiden und verstärken. Dies führt zu unerwarteten Wirkungen auf die vor Sauerstoff-Unterversorgung zu schützenden Muskulatur im Beckenbodenbereich. In Figur 6 ist lediglich schematisiert ein Muskel 25, als Strang bestehend aus einer Reihe von Muskelfasern 26 dargestellt. Der Muskel 25 wird durch eine Anzahl von zuführenden Blutgefäßen 27 mit Blut versorgt, welches in Pfeilrichtung 28 zugeführt wird, und ebenfalls sind eine Reihe von abführenden Blutgefäßen 27 schematisiert gezeigt, über die das Blut in Pfeilrichtung 29 abgeführt wird.

Es ist erkennbar, dass das starke Magnetfeld 23 die Feldlinien 24 in den Muskelfasern 26 erzeugt, wodurch diese in den Pfeilrichtungen 32 zu starken Kontraktionen angeregt werden. Dies vor allem auch dadurch, weil die vorhandenen Nervenäste 30 von den Feldlinien 24 des starken Magnetfeldes 23 stimuliert werden und hierdurch zu Kontraktionen der Muskelfasern 26 führen.

Wichtig ist nun, dass das schwächere Magnetfeld 21 mit seinen schwächeren Feldlinien 22 - in einem anderen Winkel und mit höherer Frequenz - ebenfalls auf die Nervenäste 30 des gleichen Muskels 25 wirken und diesen zusätzlich stimulieren. Damit wird eine zusätzliche Steigerung der Durchblutung der Muskelfasern 26 erreicht, weil auch die zuführenden und abführenden Blutgefäße 27 stimuliert werden und zu einer erhöhten Blutzufuhr und -abfuhr führen.

Damit wird durch die Einwirkung des zweiten, schwächeren, jedoch höherfrequenten Magnetfeldes 21 die Blutzufuhr im Muskel 25 stark gesteigert, und dieser wird aufgrund der dadurch induzierten verstärkten Durchblutung und zusätzlich durch die atemwegsgestützte, erhöhte Sauerstoffzufuhr wirksam gegen Beschädigungen geschützt, wenn er durch Einwirkung des stärkeren Magnetfeldes 21 zu Kontraktionen angeregt wird.

### Zeichnungslegende

- 1: Behandlungsstuhl
- 2: Position
- 3: Sitzteil
- 4: Rückenlehne
- 5: Magnetspule (Pelvi)
- 6: Magnetspule (QRS)
- 7: Fußteil
- 8: Bedieneinheit
- 9: Sauerstoffmaske
- 10: Leuchteneinheit
- 11: Kopfteil
- 12: Position
- 13: Position
- 14: Position
- 15: Position
- 16: Position
- 17: Linie
- 18: Linie
- 19: Position
- 20: Dauer
- 21: Magnetfeld (von 6)
- 22: Feldlinie (von 6)
- 23: Magnetfeld (von 5)
- 24: Feldlinien (von 5)
- 25: Muskel
- 26: Muskelfaser
- 27: Blutgefäß
- 28: Pfeilrichtung
- 29: Pfeilrichtung
- 30: Nervenast
- 31:
- 32: Pfeilrichtung

## Patentansprüche

1. Verfahren für das maschinengestützte invasive Beckenbodentraining der Beckenboden-Muskulatur mit der transpelvinen Magnetstimulation (TPM), bei dem über mindestens eine sitzflächenseitig angeordnete Magnetspule (5) Einzelpulse bis zu 50 Hz in den menschlichen Körper hinein erzeugt werden, um die Beckenbodenmuskulatur zu einer Kontraktion oder Entspannung anzuregen, **dadurch gekennzeichnet, dass** während der TPM-Behandlung dem menschlichen Gewebe zusätzlicher Sauerstoff zugeführt wird.

2. Verfahren nach Anspruch 1, **dadurch gekennzeichnet, dass** auch vor Beginn und nach der Abschaltung der transpelvinen Magnetstimulation (TPM) dem menschlichen Gewebe Sauerstoff zugeführt wird.

3. Verfahren nach Anspruch 1 oder 2, **dadurch gekennzeichnet, dass** die Sauerstoffzufuhr über die Atemluft erfolgt.

4. Verfahren nach einem der Ansprüche 1 bis 3, **dadurch gekennzeichnet, dass** die Atemluft in Form eines Luft-Sauerstoff oder in Form eines Luft-Sauerstoff-Ozon-Gemisches zugeführt wird.

5. Verfahren nach einem der Ansprüche 1 bis 4, **dadurch gekennzeichnet, dass** zusätzlich Wärme in die Beckenboden-Muskulatur eingetragen wird.

6. Verfahren für das maschinengestützte Beckenbodentraining der Beckenboden-Muskulatur mit der transpelvinen Magnetstimulation (TPM), bei dem über mindestens eine sitzflächenseitig angeordnete Magnetspule (5) Einzelpulse bis zu 50 Hz in den menschlichen Körper hinein erzeugt werden, um die Beckenbodenmuskulatur zu einer Kontraktion oder Entspannung anzuregen, **dadurch gekennzeichnet, dass** zur Steigerung der Durchblutung der Beckenboden-Muskulatur dem die transpelvinen Magnetstimulation (TPM) erzeugenden ersten Magnetfeld ((23) ein zweites Magnetfeld (21) höherer Frequenz und geringerer Magnetflussdichte überlagert wird.

7. Behandlungsstuhl (1) zum maschinengestützten Beckenbodentraining der Beckenboden-Muskulatur mit der transpelvinen Magnetstimulation (TPM), bei dem über mindestens eine sitzflächenseitig angeordnete Magnetspule (5) Einzelpulse bis zu 50 Hz in den menschlichen Körper hinein erzeugt werden, **dadurch gekennzeichnet, dass** am Behandlungsstuhl (1) mindestens eine Atemmaske (9) für die atemgestützte Zuführung von Sauerstoff und/oder Ozon angeordnet ist.

8. Behandlungsstuhl (1) zum maschinengestützten Beckenbodentraining der Beckenboden-Muskulatur mit der transpelvinen Magnetstimulation (TPM), bei dem über mindestens eine sitzflächenseitig angeordnete Magnetspule (5) Einzelpulse bis zu 50 Hz in den menschlichen Körper hinein erzeugt werden, **dadurch gekennzeichnet, dass** dem die transpelvinen Magnetstimulation (TPM) erzeugenden ersten Magnetfeld ((23) ein zweites Magnetfeld (21) höherer Frequenz und geringerer Magnetflussdichte überlagert ist, dessen Magnetspule (6) mindestens teilweise im Bereich der Rückenlehne (4) angeordnet ist.

9. Behandlungsstuhl nach einem der Ansprüche 7 oder 8, **dadurch gekennzeichnet, dass** mindestens eine Atemmaske (9) für die atemgestützte Zuführung von Sauerstoff und/oder Ozon angeordnet ist und dass die Magnetspule (6) zur Erzeugung des zweiten Magnetfeldes (21) mindestens teilweise im Bereich des Rückens (4) angeordnet ist.

10. Behandlungsstuhl nach einem der Ansprüche 1 bis 9, **dadurch gekennzeichnet, dass** in der Sitzfläche und/oder in der Rückenlehne zusätzliche Wärmequellen angeordnet sind.
